# EUROPEAN PATENT APPLICATION

(11) **EP 2 573 567 A1**
(43) Date of publication of application: **27.03.2013**
(21) Application number: 11783556.1
(22) Date of filing: 17.05.2011
(51) Int. Cl.: G01N 33/574, C12Q 1/48, G01N 33/573

(54) **METHOD FOR DETECTING GASTRIC CANCER**

(30) Priority: 17.05.2010 JP 2010113043
(71) Applicant: Tokyo Institute of Technology, Tokyo 152-8550 (JP); School Juridical Person The Kitasato Institute, Tokyo 108-8641 (JP)
(72) Inventor: YAMASHITA Katsuko, Yokohama-shi Kanagawa 226-8503 (JP); FUKUSHIMA Keiko, Yokohama-shi Kanagawa 226-8503 (JP); WATANABE Masahiko, Sagamihara-shi Kanagawa 252-0374 (JP); YAMASHITA Keishi, Sagamihara-shi Kanagawa 252-0374 (JP)
(74) Representative: Gille Hrabal
(86) International application number: PCT/JP2011/061330
(87) International publication number: WO 2011/145622

(57) **Abstract**

The object of the present invention is to provide a method for detecting gastric cancer, and a kit for detecting gastric cancer. The object can be solved by a method for detecting gastric cancer characterized by analyzing β1,4-N-acetylgalactosamine transferase 1. According to the present invention, gastric cancer patients can be detected at high rates, even early stage gastric cancer patients without a subjective symptom.

## Description

### TECHNICAL FIELD

The present invention relates to a method for detecting gastric cancer, characterized by analyzing β1,4-N-acetylgalactosamine transferase 1 in a liquid sample derived from a living body. The term "analyzing" or "analysis" as used herein includes a quantitative or semiquantitative measurement of the amount of a compound to be analyzed and a detection used to determine the presence or the absence of a compound to be analyzed.

### BACKGROUND ART

There are a large number of the gastric cancer patients in Asia, such as Japan, Korea, and China, and in South America. Particularly, in Japan, the mortality rate of gastric cancer is the highest of those cancers classified by organs, for long periods of time. The incidence rate and the mortality rate of gastric cancer are higher in men than in women, but the mortality rate thereof has been decreasing year after year. However, the number of deaths due to gastric cancer in 2006 is approximately 50,000. The mortality rate of gastric cancer in men is 17% which is second-worst mortality rate for a cancer classified by organ after lung cancer, and in women, it is 13%, which is the worst mortality rate for a cancer classified by organ.
Thus, the mortality rate of gastric cancer among all cancers remains high.

The gastric cancer is developed by canceration of gastric mucosa cells located on the innermost part of the stomach wall. With progression, the cancer cells invade the stomach wall and an outer serosa, and sometimes further spread to lymph nodes and other organs. The stage of gastric cancer progression is determined by invasion depth, which shows a degree of cancer invasion to the stomach wall, lymph node metastasis, metastasis to other organs, and the like, and gastric cancer is classified into four stages: stage I (IA and IB), stage II, stage III (IIIA and IIIB), and stage IV. Further, gastric cancer wherein the invasion depth is within submucosa, is sometimes referred to as an "early gastric cancer", and gastric cancer wherein the invasion depth is beyond submucosa and reaches muscularis propria deeply, is sometimes referred to as an "advanced gastric cancer".

The main treatments for gastric cancer are operative therapy, endoscopic therapy, chemotherapy, and radiation therapy. Gastric cancer patients are treated with one or a combination of above therapies, according to the progression of above clinical stages. In the above therapies, the operative therapy is the standard one and most effective.
As the operative therapy, gastric resection and a lymph node resection of particular extent (lymph node dissection) are performed. If gastric cancer patients are found in an early stage and treated with operative therapy, the many patients have a good prognosis. For example, according to the report of "Survival Rate in the Member Hospitals of the Association of Clinical Cancer Centers (Diagnosed in 1997-2000)", five year survival rate of patients in stage I exceeds 95%. Further, even in a patient of stage IV, if all of the cancer lesions may be resected, the patient can be cured.

In early gastric cancer, a lot of patients have no symptoms, and therefore it is difficult to discover gastric cancer patients by a subjective symptom thereof. Further, the earliest symptoms in gastric cancer are often a discomfort in the upper abdomen, a feeling of fullness, and the like. These symptoms are sometimes developed in chronic gastritis, gastric ulcer, and duodenal ulcer, and thus the finding of gastric cancer is often delayed. Accordingly, in order to find the gastric cancer in the early stage, it is important to find gastric cancer by a periodic health examination for gastric cancer.

Clinical examinations for gastric cancer include X-ray examination of the stomach, gastrofiberscopy, the pepsinogen test, and the Helicobacter antibody test. In particular, it is considered that the X-ray examination of the stomach is most effective to detect gastric cancer, among the clinical examinations for gastric cancer. However, it is required to drink a barium sulfate and x-radiate, thus causing a burden on subjects to be tested. Further, even if the gastric cancer is detected by X-ray examination of the stomach, the detected gastric cancer is often progressive.

Under these circumstances, the development of a clinical examination for gastric cancer capable of being carried out in health examination for gastric cancer, and the like and capable of detecting gastric cancer in the early stage, is desired. In particular, the development of gastric cancer marker capable of diagnosing gastric cancer using blood, is desired. At present, as a tumor marker which can detect gastric cancer using blood, there may be mentioned a "CEA" test and a "CA19-9" test etc. However, these tumor markers are not specific to gastric cancer. Although the detection rate of gastric cancer is increased in stage III or latter, the detection rate of early gastric cancer is 10% or less. This detection rate of gastric cancer cannot be satisfactory, and thus it is not considered that the above tumor markers are effective in health examination of gastric cancer.

### CITATION LIST

### NON-PATENT LITERATURE

[Non-patent literature 1] Taeko Dohi et al., Protein Nucleotide Enzyme, 1992(Japan), vol.37, 1868-1872
[Non-patent literature 2] Biochemica et Biophysica Acta, 2002(Netherlands), vol.1573, 356-362

### SUMMARY OF INVENTION

### TECHNICAL PROBLEM

In order to develop a tumor marker capable of detecting gastric cancer patients at an earlier stage, the present inventors have conducted intensive studies for the potential of various intracellular proteins as a tumor marker. As a result, the present inventors surprisingly found that β1,4-N-acetylgalactosamine transferase 1 (hereinafter sometimes referred to as a β4GALNT1) was detected in the blood of almost all gastric cancer patients.
The present invention is based on the above findings. Thus, the object of the present invention is to provide a method for detecting gastric cancer, and a kit for detecting gastric cancer.

The non-patent literature 1 discloses that the amount of acidic glycolipid, i.e. GM2 and GM2 synthase, activity is increased in tissues of gastric cancer compared to normal tissues of fundic gland mucosa. Further, it is reported that the GM2 synthase activity is due to β4GALNT1 of the β1,4-N-acetylgalactosamine transferase families (non-patent literature 2). However, the non-patent literature 1 discloses that GM2 is expressed in many normal tissues including stomach, i.e. the esophagus, stomach (fundic gland region and pyloric gland region), duodenum, jejunum, ileum, transverse colon, liver, lung, adrenal gland, bladder, prostate gland, testis, heart, brain, muscle, and skin. Specifically, in the adrenal gland, brain, and muscle, GM2 equivalent is expressed to the same expression level as in tissue of gastric cancer or more. Thus, GM2 synthase activity which may synthesis GM2, is not specifically observed in the tissue of gastric cancer. That is, it is considered that the above GM2 synthase activity is a glycosyltransferase activity due to β4GALNT1 expressed in the normal tissues.
Non-patent literature 1 merely discloses that the β4GALNT1 is expressed in gastric cancer tissue. Glycosyltransferase such as β4GALNT1 having GM2 synthetic activity, adds a sugar chain to a protein translated in cell cytoplasm. Therefore, in general, β4GALNT1 cannot be released to the outside of cells. In view of the above technical information, it cannot be expected from the disclosure of non-patent literature 1 that β4GALNT1 is released to the blood. However, if the β4GALNT1 is released to the blood, it is considered that the β4GALNT1 in many normal tissues, particularly the adrenal gland, brain, and muscle of a normal human, may also be released to the blood. This is because a large amount of β4GALNT1 is expressed in the normal tissues, such as the adrenal gland, brain, and muscle of normal human. In this case, it is impossible to distinguish the β4GALNT1 in blood of normal human from the β4GALNT1 in blood of gastric cancer patient. Therefore, as shown in Examples, it is surprising for those skilled in the art that the gastric cancer patients can be distinguished from normal humans by measuring the β4GALNT1 in the blood.

### SOLUTION TO PROBLEM

The present invention relates to a method for detecting gastric cancer characterized by analyzing β1,4-N-acetylgalactosamine transferase 1 in a liquid sample derived from a living body. According to a preferable embodiment of the present invention, the method is an immunological essay which comprises the steps of:
bringing an antibody specifically binding to β1,4-N-acetylgalactosamine transferase 1, or an antibody fragment having the antigen-binding site thereof, into contact with the sample to be tested; and
detecting a bound complex of the antibody, or the antibody fragment having the antigen-binding site thereof, and β1,4-N-acetylgalactosamine transferase 1.
According to a more preferable embodiment of the present invention, the liquid sample is at least one liquid sample selected from the group consisting of blood, plasma, serum, urine, saliva, sudor, and spinal fluid.
The present invention relates to a kit for detecting gastric cancer characterized by comprising an antibody specifically binding to the β1,4-N-acetylgalactosamine transferase 1, or an antibody fragment having the antigen-binding site thereof.
Further, the present invention relates to a kit for detecting gastric cancer characterized by comprising GM3 as a substrate of β1,4-N-acetylgalactosamine transferase 1.
The present specification discloses a use of the antibody specifically binding to the β1,4-N-acetylgalactosamine transferase 1, the antibody fragment having the antigen-binding site thereof, or a combination thereof, for the kit for detecting gastric cancer. Further the present specification discloses a use of the antibody specifically binding to the β1,4-N-acetylgalactosamine transferase 1, the antibody fragment having the antigen-binding site thereof, or a combination thereof, for preparing a kit for detecting gastric cancer. Furthermore the present specification discloses a use of GM3, for the kit for detecting gastric cancer, or for preparing a kit for detecting gastric cancer.

### ADVANTAGEOUS EFFECTS OF INVENTION

At present, as the health examination for gastric cancer, X-ray examination of the stomach is popularly practiced. However, it is required to drink a barium sulfate and x-radiate, and thus a burden is caused on subjects to be tested. Further, even if gastric cancer is detected by the X-ray examination of the stomach, the detected gastric cancer is often progressive. According to the detection method of the present invention, it is possible to detect gastric cancer by a blood test, and thus, the gastric cancer patients can be detected without undue stress on patients.
Further, according to the detection method of the present invention, it is possible to detect the gastric cancer patients at high rates, compared to a "CEA" test and a "CA19-9" test which are used as tumor markers capable of detecting the gastric cancer patients using blood. Specifically, the detection rates by the "CEA" test or the "CA19-9" test are 10% or less. However, almost 100% of gastric cancer patients can be detected by the detection method of the present invention.
Furthermore, the detection method of the present invention can be also used in monitoring for a recurrence of cancer after surgery, and monitoring treatment effects of irradiation therapy and chemotherapy.

### BRIEF DESCRIPTION OF THE DRAWINGS

FIG. 1 is a photograph showing that the specificities of a monoclonal antibody and a polyclonal antisera against β4GALNT1 are confirmed by an immunoprecipitation using β4GALNT1 (lane 1), β4GALNT2 (lane 2), and β4GALNT3 (lane 3), β4GALNT4 (lane 4). A: The results of immunoprecipitation to each protein using the monoclonal antibody are shown. B: The results of immunoprecipitation to each protein using the polyclonal antisera are shown.
FIG. 2 is a standard curve of β4GALNT1 by a sandwich ELISA using a monoclonal antibody and polyclonal antisera against β4GALNT1.
FIG. 3 is a graph showing the detection of β4GALNT1 in gastric cancer patients, normal humans, and other cancer patients (prostate cancer patients, ovarian cancer patients, pancreatic cancer patients, duodenal cancer patients, and esophageal cancer patients).

### DESCRIPTION OF EMBODIMENTS

### [1] Method for detecting gastric cancer

Gastric cancer is developed on gastric mucosa cells located on the innermost part of stomach wall. According to the cancer progression, gastric cancer can be classified into stage I (IA and IB), stage II, stage III (IIIA and IIIB), and stage IV. In the "Guideline for Treating Gastric Cancer" of Japanese Gastric Cancer Association, an applicable surgical therapy such as endoscopic mucosal resection (EMR), gastric reduction surgery (A, B), conventional operation, super-extended operation or the like is generally determined, according to the above clinical stages.

If gastric cancer patients are found at an early stage and treated with operative therapy, the effect of therapy is increased. For example, five years survival rate on stage IA, stage IB, stage II, stage IIIA an stage IIIB are: 87%, 93.4%, 68.3%, 50.1%, and 30.8%, respectively. Further, even on stage IV, a five years survival rate of 16.6% can be obtained.

### [1-1] Analysis of β4GALNT1

According to the detection method of the present invention, gastric cancer can be detected by analyzing β4GALT1. The β4GALNT1 belongs to the β1,4-N-acetylgalactosamine transferase families. The β4GALNT1 uses GM3 as a substrate, and transfers N-acetylgalactosamine to galactose with sialic acid by a β1→4 bond. In a normal human, the β4GALNT is expressed in the esophagus, stomach (fundic gland region and pyloric gland region), duodenum, jejunum, ileum, transverse colon, liver, lung, adrenal gland, bladder, prostate gland, testis, heart, brain, muscle, and skin, and particularly highly expressed in the adrenal gland, brain, an muscle.

In the detection method of the present invention, the method of analyzing β4GALNT1 is not particularly limited as long as the method allows detection of β4GALNT1 quantitatively or semi-quantitatively, or the method allows determination of the presence or absence of β4GALNT1. Examples of the method of analyzing β4GALNT1 include: immunological techniques using an antibody for β4GALNT1 or a fragment thereof (for example, enzyme immunoassay, latex agglutination immunoassay, chemiluminescent immunoassay, a fluorescent antibody method, radioimmunoassay, an immunoprecipitation method, an immunohistological staining method, or the western blot), biochemical techniques (for example, enzymological assay), or molecular biological assays measuring the mRNA amount of β4GALNT1. Further, the detection method of the present invention is a method for analyzing β4GALNT1 contained in a sample, derived from a living body, in vitro. By the detection method of the present invention, a full length protein of β4GALNT1 may be measured, and/or a peptide fragment of β4GALNT1 may be measured.

In the case where an immunological assay is used as the method for the analysis of β4GALNT1, a monoclonal antibody or a polyclonal antibody specifically binding to β4GALNT1 may be used. The monoclonal antibody or the polyclonal antibody can be prepared by a known method except that β4GALNT1 is used as an immunizing antigen. For example, the monoclonal antibody can be prepared according to Koehler and Milstein's method (Nature 256: 495-497, 1975). In addition, the polyclonal antibody can be prepare by conventional immunization with an antigen that is β4GALNT1 alone, or β4GALNT1 conjugated to BSA, KLH or the like, and which is mixed with an adjuvant such as Freund's complete adjuvant, for example, in the skin of a rabbit. The blood is collected when the antibody titer increases, and may be used as it is as an antiserum, or the antibody may be used after purification by a known method.
When the biochemical assay is used as the method for analyzing β4GALNT1, the enzymatic activities of β34GALNT1 can, for example, be measured in accordance with the methods of Yamashiro et al. [Yamashiro et al., J. Biol. Chem., 270, 6149-6155(1995)], whereby the amount of β4GALNT1 or the presence or absence of β4GALNT1 can be analyzed.

As an immunizing antigen for obtaining a monoclonal or polyclonal antibody against β4GALNT1, a protein consisting of an amino acid sequence of SEQ ID NO:11, or a peptide consisting of a part thereof can be used. Specifically, an antigen purified from biological samples, a recombinant antigen prepared by a genetic engineering, or a partial peptide chemically synthesized can be used. In the case of the recombinant antigen expressed by using E. coli, yeast or the like, a fusion protein fused with another protein such as SOD or TrpE can be prepared, in order to allow easy expression. In addition, a fusion protein fused with His-Tag can be prepared in order to ease purification. In particular, β4GALNT1 wherein the cytoplasmic domain and transmembrane domain are removed, is preferably expressed. For example, a polypeptide consisting of the 65th to 533th amino acids in the amino acid sequence of SEQ ID NO:11, is preferably expressed as the recombinant protein.

The antibody binding to β4GALNT1, which may be used in the method for detecting gastric cancer of the present invention, is not limited, so long as it can bind to β4GALNT1. The above antibody may be an antibody which also binds to other 1,4-N-acetylgalactosamine transferase other than β4GALNT1, such as β4GALNT2, β4GALNT3, or β4GALNT4 but preferably, an antibody which specifically binds to β4GALNT1.

In the detection method of the present invention, the above analysis is preferably an immunological assay which comprises the steps of: bringing an antibody specifically binding to β1,4-N-acetylgalactosamine transferase 1, or an antibody fragment having the antigen-binding site thereof, into contact with the sample to be tested; and detecting a bound complex of the antibody, or the antibody fragment having the antigen-binding site thereof, and β1,4-N-acetylgalactosamine transferase 1. The immunological assay includes, for example, an enzyme immunoassay, a latex agglutination immunoassay, a chemiluminescent immunoassay, a fluorescent antibody method, a radioimmunoassay, an immunoprecipitation method, an immunohistological staining method, or the western blot. As the example of the immunological assay, a sandwich immunoassay is explained below.

### [1-2] Sandwich immunoassay

When the enzyme immunoassay, chemiluminescent immunoassay, or radioimmunoassay is used as the immunological assay, the immunological assay can be performed by the following sandwich assay.

### (i) First reaction process

A capture antibody (first antibody), or an antibody fragment, binding to β4GALNT1 is immobilized to an appropriate insoluble carrier, such as a microtiter plate or a micro bead. Then the insoluble carrier is coated with an appropriate blocking agent, such as bovine serum albumin (BSA) or gelatin, to prevent a non-specific binding of the sample to the insoluble carrier. Thereafter, the sample which may contain β4GALNT1, and a first reaction buffer are added to the microtiter plate or the micro bead. Then β4GALNT1 in the sample is brought into contact with the capture antibody, to perform a reaction. Then, antigens and foreign substances that are not bound to the capture antibody (first antibody) are washed with an appropriate washing solution (for example, a phosphate buffer containing a surfactant).

### (ii) Second reaction process

Then, a labeled antibody (second antibody) in which an antibody binding to β4GALNT1 is conjugated to an enzyme such as horseradish peroxidase (HRP), is added, so as to bind the labeled antibody to the captured antigen (β4GALNT1), and form an immune complex (i.e. the capture antibody/β4GALNT1/labeled antibody complex) on the insoluble carrier such as the microtiter plate.

### (iii) Detection process

The insoluble carrier, such as the microtiter plate or the micro bead, is washed with an appropriate wash buffer, and then a colorimetric substrate or a luminescent substrate for the enzyme of the labeled antibody is added. A detectable signal may be developed by a reaction of the enzyme and the substrate. Alternatively, if the second antibody is not labeled, a labeled antibody, which may bind to the second antibody, can be used to detect the signal.

Examples of the enzyme that labels the antibody include horseradish peroxidase (HRP), alkaline phosphatase, β-galactosidase, and luciferase. Furthermore, in addition to the enzyme, luminescent substances such as acridinium derivatives, fluorescent substances such as europium, radioactive substances such as I¹²⁵, and the like may be used as a label substance. In addition, the substrate and the luminescent inducer may be properly selected in accordance with the label substance. Furthermore, the labeled antibody in the present invention may also include an antibody which is bound to a substance such as hapten or low molecular weight peptide as a detection marker, or lectin that may be used in the signal detection of the antigen-antibody reaction.

If either the first antibody or the second antibody specifically binds to the protein to be analyzed, the other antibody, which may bind to a protein other than the protein to be analyzed or may exhibit cross-reactivity to the other protein, may be used in the sandwich ELISA. In the analysis of β4GALNT1, for example, if either the first antibody or the second antibody may specifically bind β4GALNT1, the other antibody may be one which may bind to or exhibit cross-reactivity to β4GALNT2, β4GALNT3, or β4GALNT4.

Examples of the first antibody and the second antibody used in the sandwich immunoassay include, but are not particularly limited to, a polyclonal antibody, monoclonal antibody, recombinant antibody, fragment thereof or the like. As the fragment of the antibody, there may be mentioned F(ab')₂, Fab', Fab, Fv, or the like. The fragment of the antibody may be obtained by conventional methods, for example, by digesting the antibody using a protease (such as pepsin, papain, or the like) and purifying the resulting fragments by standard polypeptide isolation and purification methods.

### [1-3] Samples to be tested

In the detection method of the present invention, the samples to be tested in order to analyze for β4GALNT1, are samples of patients suspected of suffering from gastric cancer, and thus include samples of normal humans which are having a health examination for gastric cancer. In particular, the samples are liquid ones derived from the human body possibly containing β4GALNT1. Examples of the sample to be tested include: urine, blood, serum, plasma, lymph fluid, tissue fluid, spinal fluid, saliva, urine, sudor, or the like. The sample to be tested is preferably blood, serum, or plasma (hereinafter sometimes referred to as blood or the like). As shown in the Examples, β4GALNT1 is not contained in the blood, serum, and plasma of normal humans. On the other hand, β4GALNT1 is released to the blood of gastric cancer patients at the early stage of the cancer. Therefore, blood or the like is appropriate as the sample to be tested for detecting gastric cancer. These samples are used in vitro for the detection method of the present invention.

In the present invention, gastric cancer can be detected by determining the presence or absence of β4GALNT1, or determining the amount of β4GALNT1. For example, when blood or the like is used as the sample to be tested, blood is collected from a patient possibly suffering from gastric cancer, then the amount of β4GALNT1 is measured using the collected blood or serum or plasma thereof. Then it can be determined whether or not the patient has gastric cancer by comparing the amount of βGALNT1 of the patient with that of a normal human. More particularly, if the amount of β4GALNT1 in the patient is significantly higher than that of a normal human, the patient can be diagnosed with gastric cancer.
For example, in the case of after-mentioned sandwich ELISA, the average of an amount of β4GALNT1 in a normal human is calculated, and the standard deviation (SD) thereof is calculated. In the present invention, the cut-off point for detecting the gastric cancer patient is not limited, so long as gastric cancer can be detected by using the cut-off point. It is possible to define an average + SD, average + 2SD, or average + 3SD as the cut-off point, and further, it is also possible that a sample having a value higher than average is diagnosed as positive one. Preferably, the cut-off point is determined by a controlled clinical trial.

In the detection of gastric cancer of the present invention, most preferably gastric cancer is specifically detected. However, if it is necessary to diagnose gastric cancer at the early stage by a blood test in the health examination for gastric cancer, a tumor marker which can be used for preliminary screening of gastric cancer patients, is preferable. That is to say, it is preferable that the tumor marker can detect as many cancer patients as possible, among the gastric cancer patients. According to the detecting method of the present invention, as shown in Examples below, all gastric cancer patients (not including a patient supervening on other cancer) confirmed by an operation, can be detected. Therefore, the detection method of the present invention is useful as a cancer marker.

The method for detecting gastric cancer of the present invention can be used as a method for diagnosing gastric cancer, and is effective for detecting gastric cancer at an early stage in the health examination, and the like.

### [2] Kit for detecting gastric cancer

The kit for detecting gastric cancer of the present invention may contain an antibody specifically binding to β4GALNT1, the antibody fragment having the antigen-binding site thereof, or a combination thereof. As the antibody, a monoclonal antibody or a polyclonal antibody can be contained in the kit. The antibody fragment is not particularly limited, so long as it has a specific binding ability to β4GALNT1. For example, antibody fragment Fab, Fab', F(ab')₂, or Fv can be contained in the kit.
In the present specification, the wording the "antibody specifically binding to β4GALNT1" sometimes means the antibody fragment having the antigen-binding site thereof.

The detection kit of the present invention contains a desired form of antibody specifically binding to β4GALNT1, or a fragment having the antigen-binding site thereof, in accordance with the immunoassay used by the detecting kit.
For example, in the case of an immunoassay using a labeled antibody, such as enzyme immunoassay, chemiluminescent immunoassay, fluorescence antibody technique, or radioimmunoassay, the labeled antibody or antibody fragment conjugated by a labeling material can be contained in the kit. Specifically, labelling material can include peroxidase, alkaline phosphatase, β-D-galactosidase, or glucose oxidase as an enzyme; fluorescein isothiocyanate or a rare-earth metal chelate as a fluorescent material; ³H, ¹⁴C, or ¹²⁵I as a radioactive isotope; or biotin, avidin, or a chemiluminescent substance. In addition, in the case of an enzyme or a chemiluminescent substance, since they cannot develop a measurable signal by themselves, a substance corresponding to each enzyme or chemiluminescent substance is preferably contained in the kit.
In the case of the detection kit using a sandwich assay, the antibody specifically binding to β4GALNT1 which is immobilized on the insoluble carrier such as the microtiter plate or the micro bead, may be contained in the kit. Further, the kit for detecting gastric cancer of the present invention may contain β1,4-N-acetylgalactosamine transferase 1, as a standard substance. Particularly, as the standard substance, there may be mentioned β4GALNT1 purified from biological samples, β4GALNT1 prepared by a genetic engineering, or chemically-synthesized partial peptide of β4GALNT1.

The detection kit of the present invention can be used to apply the method for detecting gastric cancer characterized by analyzing β1,4-N-acetylgalactosamine transferase 1 of the present invention. The kit may contain an instruction that describes the use for detection of the gastric cancer. In addition, it may be stated on the package of the kit that the kit is for detecting gastric cancer. Furthermore, the kit for detecting gastric cancer of the present invention can be used for diagnosing gastric cancer.

The antibody specifically binding to β4GALNT1, the antibody fragment having the antigen-binding site thereof, or a combination thereof, can be used in the kit for detecting gastric cancer. Further, the antibody specifically binding to β4GALNT1, the antibody fragment having the antigen-binding site thereof, or a combination thereof, can be used for preparing the kit for detecting gastric cancer. Furthermore, the antibody specifically binding to β4GALNT1, the antibody fragment having the antigen-binding site thereof, or a combination thereof, may be contained in an agent for detecting gastric cancer.

In the case that the kit for detecting gastric cancer is one by means of enzymological assay, the kit contains GM3 as the substrate of β4GALNT1. In other words, the GM3 can be used in the kit for detecting gastric cancer, and further the GM3 can be used for preparing the kit for detecting gastric cancer.

### [3] Use of antibody specifically binding to β4GALNT1, or GM3

The antibody specifically binding to β4GALNT1 can be used in the kit for detecting gastric cancer, and further the antibody specifically binding to β4GALNT1 can be used for preparing the kit for detecting gastric cancer. As the antibody specifically binding to β4GALNT1, the antibody contained in the kit for detecting gastric cancer of the present invention, may be used. The kit for detecting gastric cancer prepared using the above antibody is one described in above item [2].

The GM3 can be used in the kit for detecting gastric cancer, and further the GM3 can be used for preparing the kit for detecting gastric cancer. As the GM3, the antibody contained in the kit for detecting gastric cancer of the present invention, may be used. The kit for detecting gastric cancer prepared using the GM3 is one described in above item [2].

### EXAMPLES

The present invention now will be further illustrated by, but is by no means limited to, the following Examples.

### <<Example of antibody preparation: Preparation of anti β4GALNT1 antibody>>

### (A) Preparation of a soluble form of β4GALNT1 (Preparation of antigen for immunization)

### (A-1) Construction of β4GALNT1 expression vector

Total RNA was purified from NUGC-4 cell lines derived from human gastric cancer, and the resulting total RNA was subjected to a reverse transcription reaction with oligo(dT)primer, to obtain cDNA. A cDNA fragment encoding a peptide lacking both cytoplasmic domain and transmembrane domain was amplified by PCR. The PCR was carried out by using the cDNA from NUGC-4 cell lines and the Forward primer for β4GALNT1 and Reverse primer for β4GALNT1 below. The obtained cDNA fragment encodes a peptide consisting of the 65th to 533th amino acids in the amino acid sequence of β4GALNT1.
Forward primer for β4GALNT1:5'-atagtcgacGTCAGGATCAAGGAGcAAGTA-3' (SEQ ID NO: 1)
Reverse primer for β4GALNT1:5'-atgaagcttCATCACTGGGAGGTCATGC-3'(SEQ ID NO: 2)
In the above sequences, the sequences in lowercase letters contain appropriate restriction sites to be fused to an expression vector. The obtained cDNAs were inserted into a cloning site of pQE9 vector (QIAGEN GmbH, Hilden, Germany), then *E. coli* M15 cells were transformed with the resulting vector. The nucleotide sequences of the obtained plasmids were determined with a Prism 310 Genetic Analyzer (Applied Biosystems, Foster City, CA).

### (A-2) Expression and purification of β4GALNT1

Preparation of recombinant β4GALNT1 was as follows. The overnight culture of transformed *E. coli* M15 cells were diluted by 1/100, added to 500mL of LB broth containing 100µg/mL ampicillin and 25µg/mL kanamycin, and grown at 37 °C for 2 hr with vigorous stirring. Isopropyl β-D-1-thiogalactopyranoside (hereafter, referred to as IPTG) having a final concentration of 1mM was added to the culture and the expression of β3Gal-T5 was induced at 37 °C for 4 hr. The *E. coli* cells were collected with centrifugation and sonicated in 20mL of Buffer A [6M guanidine-HCl, 20mM potassium phosphate buffer (pH 8.0), 10mM 2-mercaptoethanol, and 20mM imidazole]. The obtained homogenate was allowed to stand at 25 °C for 16 hr. After centrifugation at 5,000 g for 20 min, the supernatant was applied to ImL of Ni-NTA agarose (QIAGEN GmbH) and allowed to stand for 30 min with occasional mixing. The resin was packed into a plastic column and washed with 20mL of Buffer A. Thereafter, the recombinant protein was eluted with Buffer A containing 0.25M imidazole. The eluate was dialyzed against 8M urea-PBS, and concentrated with a Microcon YM-10 (Millipore Corp., Bedford, MA). Finally, 3.8mg of the recombinant protein was obtained. The obtained protein is referred to as denatured β4GALNT1 (*E. coli).*

### (B) Preparation of a soluble form of non-denatured β4GALNT1

### (Preparation of antigen for screening)

Using the same transformed *E. coli* M15 as prepared in the above item (A-1), non-denatured β4GALNT1 was prepared. The overnight culture of transformed *E. coli* M15 cells were diluted by 1/100, added to 4L of LB broth containing 100µg/mL ampicillin and 25µg/mL kanamycin, and grown at 37 °C for 2 hr with vigorous stirring. IPTG having a final concentration of 1mM was added to the culture and the expression of β3Gal-T5 was induced at 15 °C for 18 hr. The cells were collected and sonicated in 20mL of 20mM potassium phosphate buffer (pH 8.0)(containing 0.3M NaCl and 10mM imidazole). To the obtained homogenate, 2mL of 5% Triton X-100 (v/v) was added an the mixture was allowed to stand on ice for 30 min. After centrifugation, 0.5mL of Ni-NTA agarose was added to the supernatant, and the mixture was allowed to stand for 30 min with occasional mixing. The resin was packed into a plastic column an washed with 5mL of Buffer B (20mM potassium phosphate buffer (pH 8.0), 0.3M NaCl, and 20mM imidazole) containing 0.5% Triton X-100, and further washed with another 5mL of buffer B. The recombinant protein was eluted with buffer B containing 0.25M imidazole. The eluate was washed with PBS using a Microcon YM-10 (Millipore Corp., Bedford, MA) then concentrated. In the 0.76mg of proteins finally obtained, the yield of recombinant β4GALNT1 was approximately 10 %, estimated by SDS-PAGE analysis. It should be noted that this fraction contained substantial β4GALNT1 activity. The obtained protein is referred to as non-denatured β4GALNT1 (*E. coli*).

### (C) Preparation of monoclonal and polyclonal antibodies

The monoclonal and the polyclonal antibodies against β4GALNT1 were commercially prepared by Medical and Biological Laboratories Co., Ltd. (Nagoya, Japan). The monoclonal antibodies were prepared as follows. The above denatured β4GALNT1 *(E. coli)* was injected three times into the footpads of four mice at three day intervals. In accordance with the conventional method, mouse spleen cells were fused with mouse myeloma cells, then screening of hybridomas for producing antibodies was carried out using the non-denatured β4GALNT1 *(E. coli)* as the antigen. A hybridoma for producing monoclonal antibody which binds to the non-denatured β4GALNT1 was cloned. In accordance with the conventional method, the hybridoma was intraperitoneally-inoculated to five mice, to obtain 25mL of ascites. 1mL of ascitic fluid was purified with Melon gel (PIERCE Biotechnology) to obtain 3.4mg of β4GALNT1 monoclonal antibody.
In the case of the polyclonal antibody, the denatured β4GALNT1 *(E. coli)* was injected six times into a rabbit at one week intervals to obtain β4GALNT1 polyclonal antisera. 1mL of antisera was purified with Melon gel (PIERCE Biotechnology) to obtain 8.8mg of β4GALNT1 polyclonal antibody.

### (D) Identification of specificities in monoclonal and polyclonal antibodies

To identify the specificities in the resulting monoclonal and the polyclonal antibodies with respect to β4GALNT1, reactivity with respect to each of β4GALNT1, β4GALNT2, β4GALNT3, and β4GALNT4 was examined. First, each expression vector was constructed, in order to obtain cells expressing each transferase i.e. β4GALNT2, β4GALNT3, and β4GALNT4. PCR product of β4GALNT1 was obtained from cDNA derived from NUGC-4 cell lines by PCR using the primers below. PCR products of β4GALNT2, β4GALNT3, or β4GALNT4 were obtained from plasmid (these plasmids were provided from Dr. Narimatsu of the National Institute of Advanced Industrial Science and Technology) containing each full length cDNA by PCR using the primers below. The obtained PCR products were inserted into a cloning site of p3xFLAG-CMV-14 vector (Invitrogen Corporation), to construct the expression vectors. The nucleotide sequences of the obtained plasmids were determined with a Prism 310 Genetic Analyzer (Applied Biosystems, Foster City, CA).
Forward primer for β4GALNT1:5'-gctaagcttATGTGGCTGGGCCGCCGG-3' (SEQ ID NO: 3)
Reverse primer for β4GALNT1:5'-gcgtctagaCTGGGAGGTCATGCACTGC-3' (SEQ ID NO: 4)
Forward primer for β4GALNT2:5'-atataaatgcggccgcATGGGGAGCGCTGGCTTTTCC-3' (SEQ ID NO: 5)
Reverse primer for β4GALNT2:5'-tttctagaTGCGGCACATTGGAGATGGTTC-3' (SEQ ID NO: 6)
Forward primer for β4GALNT3:5'-agaattcATGGGGAGCCCCCGGGCCGC-3' (SEQ ID NO: 7)
Reverse primer for β4GALNT3:5'-gcggatatcCAGCGTCTTCATCTGGCGACG-3' (SEQ ID NO: 8)
Forward primer for β4GALNT4:5'-tttaagcttATGCCGCGGCTCCCGGTGAA-3' (SEQ ID NO: 9)
Reverse primer for β4GALNT4:5'-attctagaAGACGCCCCCGTGCGAG-3' (SEQ ID NO: 10)

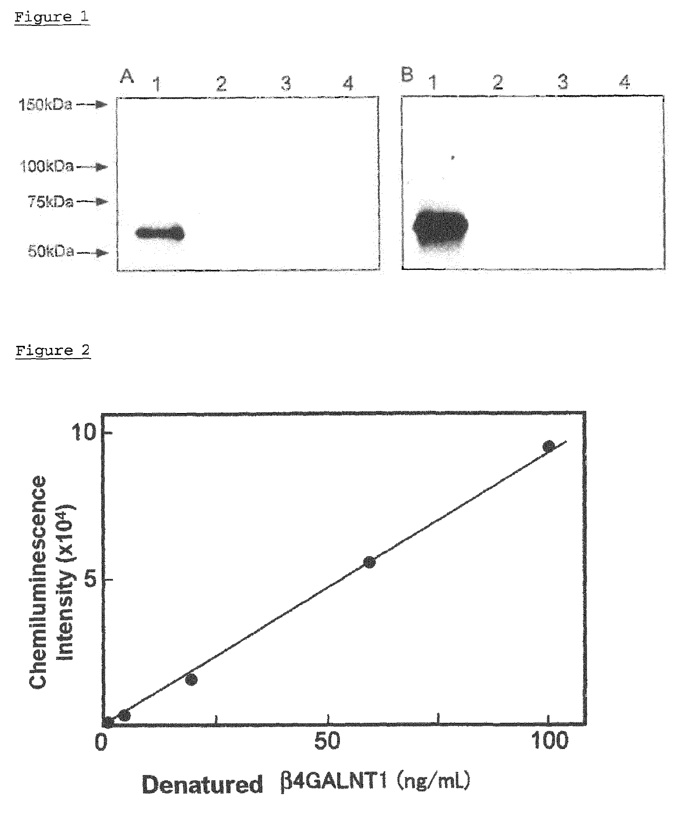

Cos cells were transfected with the each expression vector using a Lipofectamine (transfection agent; Invitrogen Corporation).
Next, the reactivity of the antibody against each transferase was identified by using immunoprecipitation. Each expressed transferase was immunoprecipitated from the resulting transfected cells using the monoclonal antibody or the polyclonal antibody. The resulting immunoprecipitates were loaded with polyacrylamide gel, and transferred to a nitrocellulose membrane. After blocking, the membrane was incubated with anti FLAG antibody (1µg/mL: Sigma). The membrane was washed, and further incubated with HRP labeled anti mouse IgG/IgM rabbit antibody (0.3µg/mL: Jackson ImmunoReseach). The chemiluminescence detection was carried out using ECL Western blot detection reagent (GE Healthcare).
As shown in Fig. 1, the monoclonal antibody and the polyclonal antibody showed an intense reactivity to β4GALNT1, but did not show reactivity to the other transferases.

### <<Example 1: Construction of sandwich ELISA of β4GALNT1>>

An immunoassay system for β4GALNT1 by a sandwich ELISA method was constructed using the monoclonal antibody and polyclonal antibody obtained by immunizing β4GALNT1.
The β4GALNT1 polyclonal antibody was diluted to a concentration of 1mg/mL with 25mM MES buffer (pH6.0), and immobilized on magnetic beads (Dynabeads MyOne Carboxylic acid: Invitrogen Corporation). The resulting magnetic beads were suspended in PBS, to prepare a suspension of a concentration of 5mg/mL. The β4GALNT1 monoclonal antibody was labeled with alkaline phosphatase using an Alkaline phosphatase labeling kit (Cosmo Bio Co., Ltd.)

A diluent for diluting a standard substance was prepared by adding 50µg of the β4GALNT1 polyclonal antibody-immobilized magnetic beads to normal human sera diluted with PBS by a factor of 10, incubating for 1 hour, and removing the magnetic beads therefrom. The denatured β4GALNT1 (*E*. *coli*), as the standard substance, was diluted to a concentration of 200ng/mL, 50ng/mL, and 12.5ng/mL with the diluent for diluting the standard substance. To 50µL of the resulting diluents, 50µg of the antibody-immobilized beads were added, and the whole was incubated at 25°C for 2 hour. After washing with PBS-T three times, 50µL of β4GALNT1 monoclonal antibody labeled with alkaline phosphatase (1µg/mL) was added and incubated at 25°C for 1hr. The wells were washed with PBS-T four times, and 100µL of BM Chemiluminescence ELISA Substrate (Roche) was added and chemiluminescent quantification was carried out by using a Plate CHAMELEON V (HIDEX Oy, Turku, Finland).
As shown in Figure 2, a good quantitative standard curve of β4GALNT1 was obtained.

### <<Example 2: Measurement in sera of gastric cancer patients using the β4GALNT1 detection system>>

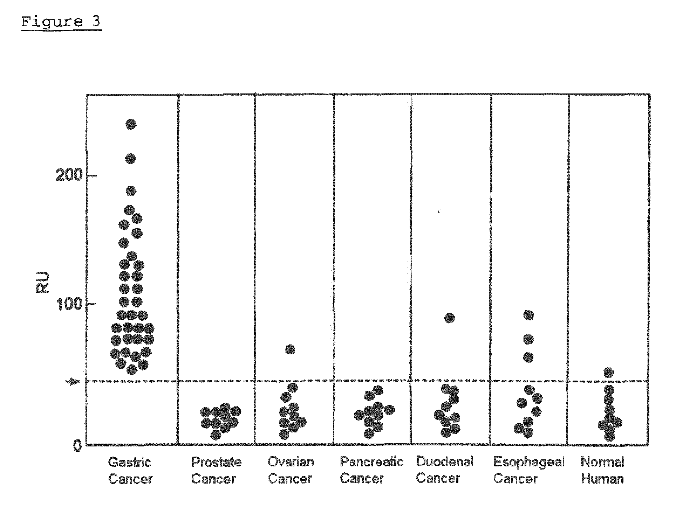

Sera of 60 gastric cancer patients, sera of 10 samples of healthy adult humans, and sera of 50 other cancer patients (prostate cancer patients, ovarian cancer patients, pancreatic cancer patients, duodenal cancer patients, and esophageal cancer patients) were measured using the β4GALNT1 detection system. The sera of the gastric cancer patients to be used in the measurement were obtained from gastric cancer patients of stages I to IV, confirmed by abdominal operation. Any cancer developments other than gastric cancer were not confirmed in these gastric cancer patients.
The procedure of ELISA used in Example 1 was repeated, except that the sera of patients and of healthy humans diluted with PBS-T by a factor of 10 were used instead of denatured β4GALNT1 (*E*. *coli*). The amount of β4GALNT1 in the sera was determined by the standard curve in Figure 2. The value of the average in healthy humans + 2SD was defined as a cut-off point, and it was determined whether the serum of each patient was positive or negative. The results were shown in Figure 3.

The values of β4GALNT1 in bloods of gastric cancer patients were high, compared to those of normal humans. Therefore, it is possible for the gastric cancer patients to be almost completely distinguished from the normal humans.

### <<Comparative Example 1: Measurement of CEA in sera of gastric cancer patients>>

In this Example, the amount of CEA, which is a conventional marker for gastric cancer, was measured, in sera of 25 of the 60 gastric cancer patients measured in Example 2.
The measurement of CEA was carried out using the kit of "LUMIPULSE Presto CEA" (FUJIREBIO Inc.) in accordance with the protocol attached thereto. As shown in Table 1, the positive rate for CEA in gastric cancer patients was 8% (2/25).

### <<Comparative Example 2»

In this Example, the amount of CA19-9, which is a conventional marker for gastric cancer, was measured, in sera of 25 of the 60 gastric cancer patients measured in Example 2.
The measurement of CA19-9 was carried out using the kit of "LUMIPULSE Presto CA19-9" (FUJIREBIO Inc.) in accordance with the protocol attached thereto. As shown in Table 1, the positive rate for CEA in gastric cancer patients was 4% (1/25).

**Table 1**

| Marker | Positive | Negative | Positive Rate |
|---|---|---|---|
| CEA | 2 | 23 | 8% |
| CA19-9 | 1 | 24 | 4% |

### INDUSTRIAL APPLICABILITY

Almost 100% of gastric cancer patients can be detected by the method for detecting gastric cancer and the kit for detecting gastric cancer of the present invention. Therefore, the method and the kit of the present invention can be used in health examination for gastric cancer.
Although the present invention has been described with reference to specific embodiments, various changes and modifications obvious to those skilled in the art are possible without departing from the scope of the appended claims.

## Claims

1. A method for detecting gastric cancer **characterized by** analyzing β1,4-N-acetylgalactosamine transferase 1 in a liquid sample derived from a living body.

2. The method for detecting gastric cancer according to claim 1, wherein the method is an immunological assay which comprises the steps of:
bringing an antibody specifically binding to β1,4-N-acetylgalactosamine transferase 1, or an antibody fragment having the antigen-binding site thereof, into contact with the sample to be tested; and
detecting a bound complex of the antibody, or the antibody fragment having the antigen-binding site thereof, and β1,4-N-acetylgalactosamine transferase 1.

3. The method for detecting gastric cancer according to claim 1 or 2, wherein the liquid sample is at least one liquid sample selected from the group consisting of blood, plasma, serum, urine, saliva, sudor, and spinal fluid.

4. A kit for detecting gastric cancer **characterized by** comprising an antibody specifically binding to the β1,4-N-acetylgalactosamine transferase 1, or an antibody fragment having the antigen-binding site thereof.

5. A kit for detecting gastric cancer **characterized by** comprising GM3 as a substrate of β1,4-N-acetylgalactosamine transferase 1.

6. A use of an antibody specifically binding to the β1,4-N-acetylgalactosamine transferase 1, or an antibody fragment having the antigen-binding site thereof, for preparing a kit for detecting gastric cancer.

7. A use of GM3 as a substrate of β1,4-N-acetylgalactosamine transferase 1, for preparing a kit for detecting gastric cancer.


SEQUENCE LISTING

<110>  TOKYO INSTITUTE OF TECHNOLOGY
       THE KITASATO INSTITUTE

<120>  Method of detecting stomach cancer

<130>  TIT-876

<150>  JP 2010-113043
<151>  2010-05-17

<160>  11

<170>  PatentIn version 3.1

<210>  1
<211>  30
<212>  DNA
<213>  Artificial

<220>
<223>  Forward primer for b3Gal-T5

<400>  1
atagtcgacg tcaggatcaa ggagcaagta                                        30


<210>  2
<211>  28
<212>  DNA
<213>  Artificial

<220>
<223>  Reverse primer for b3Gal-T5

<400>  2
atgaagcttc atcactggga ggtcatgc                                          28


<210>  3
<211>  27
<212>  DNA
<213>  Artificial

<220>
<223>  Forward primer for b4GALNT1

<400>  3
gctaagctta tgtggctggg ccgccgg                                           27


<210>  4
<211>  28
<212>  DNA
<213>  Artificial

<220>
<223>  Reverse primer for b34GALNT1

<400>  4
gcgtctagac tgggaggtca tgcactgc                                          28


<210>  5

<211>  37
<212>  DNA
<213>  Artificial

<220>
<223>  Forward primer for  3Gal-T1

<400>  5
atataaatgc ggccgcatgg ggagcgctgg cttttcc                                37


<210>  6
<211>  30
<212>  DNA
<213>  Artificial

<220>
<223>  Reverse primer for  3Gal-T1

<400>  6
tttctagatg cggcacattg gagatggttc                                        30


<210>  7
<211>  27
<212>  DNA
<213>  Artificial

<220>
<223>  Forward primer for  3Gal-T2

<400>  7
agaattcatg gggagccccc gggccgc                                           27


<210>  8
<211>  30
<212>  DNA
<213>  Artificial

<220>
<223>  Reverse primer for  3Gal-T2

<400>  8
gcggatatcc agcgtcttca tctggcgacg                                        30


<210>  9
<211>  29
<212>  DNA
<213>  Artificial

<220>
<223>  Forward primer for  3GalNAc-T1

<400>  9
tttaagctta tgccgcggct cccggtgaa                                         29


<210>  10
<211>  25
<212>  DNA
<213>  Artificial

<220>

<223>  Reverse primer for  3GalNAc-T1

<400>  10
attctagaag acgcccccgt gcgag                                              25


<210>  11
<211>  533
<212>  PRT
<213>  Homo sapiens

<400>  11